# FASCICULE DE BREVET EUROPEEN

(11) **EP 2 459 526 B1**
(45) Date de publication et mention de la délivrance du brevet: **26.06.2013**
(21) Numéro de dépôt: 10752893.7
(22) Date de dépôt: 29.07.2010
(51) Int. Cl.: C07C 315/06, C07C 317/04, C07C 319/26, C07C 321/14

(54) **COMPOSITION À BASE DE SULFURE ORGANIQUE À ODEUR MASQUÉE**
ORGANISCHE SULFIDZUSAMMENSETZUNG MIT MASKIERTEM GERUCH
ORGANIC-SULPHIDE COMPOSITION WITH MASKED ODOUR

(30) Priorité: 31.07.2009 FR 0955398; 18.08.2009 US 234680 P
(43) Date de publication de la demande: 06.06.2012
(73) Titulaire: Arkema France, 92700 Colombes (FR)
(72) Inventeur: SCHMITT, Paul-Guillaume, F-64230 Lescar (FR)
(74) Mandataire: Lhoste, Catherine
(86) Numéro de dépôt international: PCT/FR2010/051607
(87) Numéro de publication internationale: WO 2011/012815

(56) Documents cités:
- EP-A1- 0 933 358
- EP-A1- 0 976 726
- US-A- 5 559 271

## Description

La présente invention concerne le domaine des sulfures organiques et plus particulièrement celui des sulfures d'alkyle ou de dialkyle, notamment du sulfure de diméthyle (ou diméthyldisulfure, ou encore DMDS), ainsi que celui de leurs oxydes, et notamment du diméthylsulfoxyde (ou DMSO).

Il est bien connu que les sulfures organiques présentent en général une odeur forte, désagréable, voire agressive. En particulier, le DMDS présente une odeur forte et agressive due en partie à la présence d'impuretés fortement odorantes et en partie à l'odeur aillée et éthérée intrinsèque au DMDS. Il en est de même pour la plupart des sulfures organiques. De manière générale, les oxydes de ces sulfures organiques, en particulier le DMSO, présentent une odeur moins agressive, mais toutefois, suivant les concentrations en impuretés, cette odeur peut être désagréable, et gênante pour l'utilisateur final.

Cette forte odeur entrave le développement accru de ces produits, par exemple dans le cas du DMDS, dans des applications telles que la sulfuration de catalyseurs ou en tant qu'additif de charge pour le vapocraquage. Pourtant, par rapport à d'autres produits utilisés dans ces applications tels que les *tertio-*alkylpolysulfures, le DMDS présente de nombreux avantages, notamment une teneur en soufre élevée (68 %) et des produits de dégradation non cokants (CH₄, H₂S). De plus, dans ces applications, le DMDS conduit à des performances généralement supérieures aux autres produits tels que les *tertio*-alkylpolysulfures.

Cependant, ces autres produits peuvent avoir des niveaux odorants inférieurs à celui du DMDS et peuvent de ce fait rendre leur utilisation privilégiée dans certains cas.

Il existe aujourd'hui de nombreux procédés de synthèse de sulfures organiques et de leurs oxydes. Les plus utilisés, et les plus rentables d'un point de vue industriel, présentent toutefois le désavantage de conduire à des sous-produits responsables des odeurs désagréables des produits finaux.

Par exemple, parmi les méthodes de synthèse du DMDS, une méthode particulièrement efficace et économique est l'oxydation du méthylmercaptan par le soufre selon la réaction :

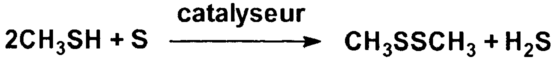

Cette oxydation du méthylmercaptan par le soufre, catalysée par des agents basiques organiques ou inorganiques, homogènes ou hétérogènes, en discontinu ou en continu, s'accompagne d'un dégagement d'hydrogène sulfuré ainsi que de polysulfures de diméthyle (CH₃SₓCH₃) de rang en soufre x supérieur à 2.

Pour fabriquer le DMDS selon ce procédé avec des rendements élevés et une production limitée en DMPS (polysulfures de diméthyle de rang supérieur à 2), le brevet EP 0 446 109 dont le contenu est incorporé ici par référence décrit un procédé de préparation comprenant deux zones de réaction interrompues par une zone de dégazage intermédiaire et suivies d'une zone de distillation. Bien que performant en termes de rendement et de sélectivité en DMDS, il s'avère que ce procédé conduit à laisser dans le produit fini une quantité non négligeable de méthylmercaptan (MM, environ 4000 ppm) ainsi qu'une très faible quantité de sulfure de diméthyle (DMS, environ 300 ppm) provenant du méthylmercaptan utilisé ou produit lors de la synthèse du DMDS.

La résultante de ces impuretés volatiles est qu'elles conduisent à rendre très désagréable et agressive l'odeur du DMDS et cette forte odeur est considérée comme une gêne importante lors de la manipulation de ce produit par les utilisateurs.

Pour masquer l'odeur de polysulfures organiques, le brevet US 5 559 271 préconise de leur ajouter une certaine quantité de produit masquant tel que, notamment, la vanilline ou l'éthylvanilline. Bien que sa formule générale inclue le DMDS, ce brevet vise plus particulièrement le traitement des polysulfures lourds comme, par exemple, le pentasulfure de di-*tert*-nonyle. L'application de cette méthode au DMDS ne permet pas de masquer efficacement son odeur désagréable.

Le brevet EP 0 976 726 décrit que, dans le cas spécifique du DMDS présentant des teneurs réduites en impuretés volatiles fortement odorantes telles que le méthylmercaptan et le sulfure de diméthyle, les agents masquant d'odeur les plus efficaces sont choisis parmi les esters de formule générale R¹CO₂R² dans laquelle R¹ représente un radical hydrocarboné, linéaire ou ramifié, éventuellement insaturé, contenant de 1 à 4 atomes de carbone, et R² représente un radical hydrocarboné contenant de 2 à 8 atomes de carbone, linéaire, ramifié ou cyclique, éventuellement insaturé.

Les solutions connues aujourd'hui pour masquer les odeurs des sulfures organiques et leurs oxydes ne sont cependant pas entièrement satisfaisantes, et il est constamment recherché des masquants d'odeur pour ces produits qui soient plus efficaces, et en particulier dont l'odeur ressentie par l'utilisateur final soit la plus agréable possible.

Ainsi l'invention a, entre autres, pour objet une composition comprenant :
a) au moins un sulfure organique, éventuellement sous forme oxyde, de formule générale (1) : 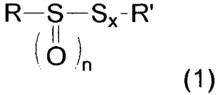 dans laquelle R est choisi parmi un radical alkyle, linéaire ou ramifié, contenant de 1 à 4 atomes de carbone, et un radical alcényle, linéaire ou ramifié, contenant de 2 à 4 atomes de carbone ; n est égal à 0, 1 ou 2 ; x est un nombre entier choisi parmi 0, 1, 2, 3 ou 4, de préférence x représente 1, 2, 3 ou 4 ; R' est choisi parmi un radical alkyle, linéaire ou ramifié, contenant de 1 à 4 atomes de carbone, et un radical alcénylène, linéaire ou ramifié, contenant de 2 à 4 atomes de carbone ou, seulement lorsque n = x = 0, un atome d'hydrogène ; et
b) au moins un agent masquant d'odeur comprenant au moins un mono-ester, au moins un di- et/ou tri-ester, au moins un alcool, au moins une cétone et éventuellement au moins un terpène.

Selon un mode de réalisation préféré, l'agent masquant d'odeur comprend :
b1) de 1 % à 40% en poids d'au moins un mono-ester ;
b2) de 10% à 70% en poids d'au moins un di- et/ou tri-ester ;
b3) de 1 % à 30% d'au moins un alcool ;
b4) de 0,5% à 20% d'au moins une cétone de formule R^{a}-CO-R^{b}, dans laquelle R^{a} représente une chaîne hydrocarbonée comportant de 1 à 6 atomes de carbone, linéaire ou ramifié, éventuellement comprenant une ou plusieurs insaturation(s) sous forme de double(s) liaison(s), et R^{b} représente une chaîne hydrocarbonée cyclique ou bien une chaîne hydrocarbonée linéaire ou ramifiée éventuellement mais de préférence substituée par une structure cyclique, R^{b} comportant de 6 à 12 atomes de carbone, comprenant éventuellement une ou plusieurs insaturation(s) sous forme de double(s) liaison(s) et étant éventuellement substitué par un ou plusieurs groupes hydroxyle ; et
b5) éventuellement jusqu'à 20% d'au moins un terpène.

Dans la description de la présente invention, les pourcentages sont indiqués en poids, sauf mention spécifique contraire. Les pourcentages de b1, b2, b3, b4 et b5 sont des pourcentages en poids exprimés par rapport au poids total de l'agent masquant d'odeur b). Sauf mention contraire, « ppm » signifie partie par million en poids.

Selon un mode de réalisation, le composant a) utilisé dans la composition selon la présente invention est un sulfure organique, éventuellement sous forme d'oxyde, obtenu selon tout procédé connu en soi, ou encore disponible dans le commerce, et de préférence à teneur réduite en impuretés volatiles. De telles impuretés sont par exemple le méthylmercaptan (MM) et le sulfure de diméthyle (DMS) dans le cas du DMDS ; en ce qui concerne le DMSO, les impuretés les plus souvent rencontrées sont par exemple le DMS, le DMDS et/ou le BMTM (bis(méthylthio)méthane, également connu sous le nom de 2,4-dithiapentane).

Toute méthode connue de l'homme du métier destinée à éliminer, ou tout au moins réduire, les impuretés volatiles précitées peut convenir, parmi lesquelles on peut citer, de manière non limitative la distillation, l'évaporation sous courant de gaz inerte tel que azote, air, et autres.

En particulier, les quantités de MM et de DMS présentes dans le DMDS peuvent avantageusement être fortement diminuées par distillation. Cette méthode présente l'avantage d'éliminer conjointement le MM et le DMS alors que les méthodes habituelles de réduction d'odeur sont généralement basées sur l'élimination des mercaptans résiduels par réaction spécifique de la fonction mercaptan avec un agent d'élimination tel qu'une base ou un oxyde d'alcène en présence d'une base. Ces méthodes seraient sans effet sur le DMS présent dans le DMDS.

Selon un mode de réalisation préféré, les teneurs résiduelles en MM et DMS, après étêtage du DMDS, ne dépassent pas 100 ppm et 50 ppm, respectivement, en poids par rapport au DMDS. La teneur résiduelle en méthylmercaptan (MeSH) dans le DMDS ne devrait pas dépasser les 500 ppm en poids.

Dans le cas du DMSO, les teneurs en impuretés, telles DMS, DMDS et/ou BMTM, doivent avantageusement être inférieures 100 ppm, de préférence inférieures à 50 ppm, de préférence encore inférieures à 10 ppm, pour chacune des impuretés prises séparément.

Dans un mode de réalisation, le composant a) de la composition selon la présente invention répond à la formule (1 a) : 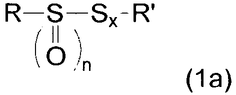 dans laquelle R et R', identiques ou différents, sont choisis parmi un radical alkyle, linéaire ou ramifié, contenant de 1 à 4 atomes de carbone, et un radical alcényle, linéaire ou ramifié, contenant de 1 à 4 atomes de carbone ; n est égal à 0 ; et x est un nombre entier choisi parmi 1, 2, 3 ou 4, de préférence 2, 3 ou 4.

De préférence le composant a) de formule (1a) est le DMDS.

Dans un autre mode de réalisation, le composant a) de la composition selon la présente invention répond à la formule (1 b) : 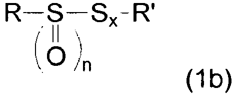 dans laquelle R et R', identiques ou différents, sont choisis parmi un radical alkyle, linéaire ou ramifié, contenant de 1 à 4 atomes de carbone, et un radical alcényle, linéaire ou ramifié, contenant de 1 à 4 atomes de carbone ; n est égal à 0, 1 ou 2 ; et x est égal à 0.

De préférence le composant a) de formule (1 b) est le DMSO.

Selon l'invention, le masquage de l'odeur du sulfure organique ou des sulfures organiques, et/ou de leur(s) forme(s) oxyde(s), répondant à la formule (1) précédemment décrite, est obtenu, par ajout auxdits sulfures ou oxydes, une composition définie sous b) précédemment.

La présente invention présente l'avantage de masquer l'odeur désagréable d'au moins un sulfure organique et/ou de son(ses) oxyde(s), sans en modifier chimiquement la nature. Ainsi, la présente invention propose une composition comprenant a) une quantité majoritaire d'au moins un sulfure organique de formule (1) définie précédemment, à laquelle est ajoutée une quantité minoritaire d'une composition b) masquant l'odeur désagréable du composant a).

La composition à odeur masquée selon la présente invention peut être préparée selon tout procédé connu en soi en combinant simplement au moins un composant a) avec au moins une composition b) de masquage d'odeur. On peut par exemple ajouter au moins une composition b) à au moins un composant a), ou vice-versa, éventuellement sous agitation et/ou éventuellement en chauffant. Plus généralement, toute méthode connue de mélange et/ou de chauffage peut être utilisée.

La préparation de la composition selon l'invention peut par exemple être effectuée sous pression atmosphérique, à une température comprise entre 0°C et 100°C, de préférence comprise entre la température ambiante et environ 80°C. La préparation peut également être effectuée sous pression ou sous dépression, à des températures comprises dans les gammes indiquées ci-dessus.

La période de temps requise pour la préparation de la composition à odeur masquée selon l'invention varie selon la nature et la quantité du ou des composant(s) a) et de la ou des composition(s) b), mais aussi en fonction de la température et de la pression choisies. En règle générale, cette durée correspond à la durée nécessaire pour obtenir un mélange homogène et produisant l'effet recherché de masquage d'odeur du ou des composant(s) a) ; elle est généralement comprise entre quelques secondes et quelques minutes, voire une ou plusieurs heures.

Le procédé de préparation mentionné ci-dessus peut être réalisé par lots (procédé « batch ») ou encore en continu.

La composition b) est mélangée, selon toute méthode connue de l'homme du métier, au composant a) en une quantité allant de quelques ppm, par exemple 10 ppm, à 2%, de préférence de 10 ppm à 1 % en poids par rapport au poids total de la composition. La quantité d'agent masquant (composition b)) peut varier dans de grandes proportions dans la plage indiquée ci-dessus, selon l'effet souhaité, l'intensité de l'odeur à masquer, les teneurs résiduelles respectives des diverses impuretés pouvant être présentes dans le(s) composant(s) a) précédemment définis, et autres.

Des quantités d'agent masquant inférieures à quelques ppm peuvent être trop faibles pour obtenir l'effet souhaité. Des quantités d'agent masquant supérieures à 2% peuvent avoir des effets néfastes selon les applications visées pour les sulfures et/ou oxydes organiques.

Dans le cas du DMDS par exemple, un des principaux avantages de ce sulfure organique est la teneur en élevée soufre (68%). Une teneur trop importante d'agent masquant dans la composition conduirait à baisser ce titre en soufre et diminuerait l'intérêt de ce produit dans ses principales applications.

De préférence, et à titre d'exemple non limitatif, la teneur en agent(s) masquant d'odeur b) est comprise entre 0,001% et 0,5% en poids par rapport au poids total de la composition, et plus particulièrement comprise entre environ 0,1% et 0,5% en poids, avantageusement égale à environ 0,3%, notamment lorsque le sulfure organique dont on souhaite masquer l'odeur est le DMDS.

Également à titre d'exemple non limitatif, dans le cas du DMSO, la teneur maximale en agent(s) masquant d'odeur est avantageusement comprise entre 0,001% et 0,2% en poids par rapport au poids total de la composition, de préférence comprise entre 100 ppm et 1000 ppm, par exemple environ 500 ppm en poids.

Comme indiqué précédemment, la composition selon la présente invention comprend au moins une composition d'agent masquant d'odeur b), ledit agent comprenant de 1 % à 40%, de préférence de 2% à 35%, de préférence encore de 5% à 30% en poids, par rapport au poids total de la composition b), d'au moins un mono-ester mentionné sous b1).

Comme exemples illustratifs, mais non limitatifs, de mono-esters mentionnés sous b1), on peut citer les esters d'acides en C₂-C₂₀ saturés ou insaturés, tels que les acétates, propionates, butyrates, méthylbutyrates, pentanoates, hexanoates, heptanoates, caproates, oléates, linoléates, linolénates, d'éthyle, de propyle, de butyle, de pentyle, de 2-méthylbutyle, d'iso-amyle, d'hexyle, de benzyle, de phényléthyle, de menthyle, de carvyle, et autres, ainsi que leurs mélanges.

Sont plus particulièrement préférés l'acétate d'iso-amyle, l'acétate d'hexyle, le butyrate de 2-méthylbutyle, le butyrate d'iso-amyle, l'acétate de benzyle, l'acétate de phényléthyle et les mélanges de ces composés.

La composition d'agent masquant d'odeur b) comprend également au moins un di- et/ou tri-ester b2), en une quantité comprise entre 10% et 70% en poids, de préférence entre 15% et 65% en poids, de préférence encore entre 20% et 60% en poids, tel que, de manière non limitative, au moins un di- et/ou tri-ester choisi(s) parmi les *ortho*-phtalates, tel que l'*ortho*-phtalate de diéthyle ; les citrates, tels que le citrate de triéthyle ; et les malonates, tel que le malonate de diéthyle.

L'agent masquant d'odeur b) comprend également de 1% à 30%, de préférence de 5% à 25% en poids par rapport au poids total de la composition, d'au moins un alcool b3), avantageusement au moins un mono-alcool comprenant de 1 à 30 atomes de carbone, de préférence de 6 à 20 atomes de carbone, de préférence encore de 8 à 11 atomes de carbone, lesdits atomes de carbone formant une chaîne linéaire ou ramifiée comportant éventuellement une ou plusieurs insaturation(s) sous forme de double(s) liaison(s), et comportant éventuellement une structure cyclique à 5 ou 6 chaînons, saturée, ou totalement ou partiellement insaturée.

Les alcools précédemment définis sont de préférence des monoalcools, la fonction hydroxyle étant de préférence portée par un atome de carbone sp². Il doit être entendu que la fonction hydroxyle peut également être portée par un atome de carbone inclus dans une structure cyclique comme définie précédemment.

Les alcools utilisés dans l'agent masquant d'odeur et tels que définis ci-dessus sont avantageusement et à titre d'exemples non limitatifs choisis parmi le menthol, le *néo*-menthol, l'alcool phényléthylique, l'alcool benzylique, le citronellol, le dihydromyrcénol, le dihydrotérpinéol, le dimétol, l'éthyl-linalol, le géraniol, le linalol, le tétrahydrolinalol, le tétrahydromyrcénol, le nérol, et autres, ainsi que les mélanges de deux ou plusieurs d'entre eux.

La cétone ou les cétones indiquées sous b4) précédemment sont choisies, à titre d'exemples non limitatifs, et de préférence, parmi les damascones, les damascénones, les ionones, les irisones, les méthylionones, la frambinone (n° CAS 5471-51-2), et autres, ainsi que les mélanges de celles-ci. La quantité de cétone(s) est avantageusement comprise entre 0,5% et 20%, de préférence entre 1 % et 10% en poids par rapport au poids total de la composition.

L'agent masquant d'odeur peut éventuellement comprendre également jusqu'à 20%, de préférence de 1 % à 10% en poids par rapport au poids total de la composition, d'au moins un terpène.

Comme exemples de terpènes, indiqués sous b5), qui peuvent être utilisés, on peut citer, de manière non limitative, les terpinènes, le myrcène, le limonène, le terpinolène, les pinènes, le sabinène, le camphène, et autres, les mélanges de deux ou plusieurs d'entre eux, ainsi que les essences à base de terpènes, notamment celles comprenant ces ingrédients.

En outre, l'agent masquant d'odeur utilisable dans le cadre de la présente invention peut comprendre, en quantités minoritaires, d'autres agents (fragrances) habituellement utilisés dans le domaine de la parfumerie, et en particulier un ou plusieurs composés porteurs de fonction(s) aldéhyde et/ou cétone cyclique, parmi lesquels on peut citer, de manière non limitative, le géranial, le néral, le citronellal, la menthone, l'iso-menthone, le 1,8-cinéole, l'ascaridole, la flavonone, et leurs mélanges.

La composition b) destinée à maquer l'odeur des sulfures organiques, et telle que décrite précédemment peut, le cas échéant, voir si nécessaire, comprendre en outre un ou plusieurs additifs couramment utilisés dans le domaine. De tels additifs peuvent par exemple être choisis parmi, et de manière non limitative, les solvants, les pigments, les colorants, les conservateurs, les biocides, et autres.

Parmi les solvants, des exemples tout particulièrement préférés sont les alcools, les éthers, les esters et les glycols. De manière particulièrement avantageuse, le solvant est choisi parmi le phtalate de diéthyle, l'éthylène glycol, le propylène glycol, le di-éthylène glycol, le dipropylène glycol, les poly-éthylène glycols, les polypropylène glycols, et leurs mélanges, et de manière encore plus avantageuse parmi le phtalate de diéthyle, le dipropylène glycol, et leurs mélanges.

Il doit être compris que un mono-, di- ou tri-ester présent dans la composition b) d'agent masquant d'odeur, en tant que composant b1) et ou b2) peut également avoir les fonctions des solvants définis ci-dessus.

Une composition typique d'agent masquant d'odeur adaptée pour les sulfures organiques et leurs formes oxydes, selon la présente invention comprend en poids :
- de 5% à 30% en poids d'au moins un mono-ester b1), choisi parmi l'acétate d'iso-amyle, le méthyl-2-butyrate d'ethyle, le butyrate d'*iso*-amyle, l'acétate de phényléthyle, le caproate d'éthyle, l'acétate de benzyle, l'acétate d'hexyle et leurs mélanges ;
- de 20% à 60% en poids d'au moins un di- et/ou tri-ester b2) choisi(s) parmi les *ortho*-phtalates, tel que l'*ortho*-phtalate de diéthyle ; les citrates, tels que le citrate de triéthyle ; et les malonates, tel que le malonate de diéthyle, et leurs mélanges ;
- de 5% à 25% d'au moins un alcool, de préférence d'au moins deux alcools, de préférence encore d'au moins trois alcools, tels que décrits précédemment sous b3) ;
- de 1 % à 10% d'au moins une cétone, de préférence au moins deux cétones, de préférence encore au moins trois cétones, telles que décrites précédemment sous b4) ; et
- de 1% à 10% d'au moins un, de préférence au moins deux, de préférence un mélange de, terpènes référencés plus haut sous b5).

Cette composition, notée C*ᵢ* dans la suite du présent exposé, est tout particulièrement adaptée pour le masquage de l'odeur, pour l'amélioration de l'odeur, du DMDS. Cette même composition C*ᵢ* peut également être avantageusement utilisée pour masquer, améliorer, l'odeur du DMSO.

Un exemple représentatif, mais non-limitatif, d'une telle composition C*ᵢ* est reproduite ci-dessous, dans laquelle chacun des composants comprend un, plusieurs, voire tous les composés listés :

| | |
|---|---|
| *Composant b1)* | 16,00% |
| comprenant acétate de benzyle, acetate d'hexyle, acétate d'iso-amyle, acétate de phényléthyle, caproate d'ethyle, méthyl-2-butyrate d'ethyle | |
| *Composant b2)* | |
| comprenant malonate de diéthyle, phtalate de diéthyle | 50,00% |
| *Composant b3)* | |
| comprenant alcool phényléthylique, citronellol, géraniol, linalol, cis-3-hexènol | 20,60% |
| *Composant b4)* | |
| comprenant 1-(4-hydroxyphenyl) butane-3-one, *alpha*-irisone | 4,50% |
| *Composant b5)* | |
| terpènes d'orange | 7,00% |
| *Autres* | |
| comprenant citral, éthylmaltol, éthylméthyl phénylglycidate | 1,90% |

Ces compositions sont données à titre d'exemples et n'ont rien de restrictif quant à la diversité potentielle de compositions que permet la présente invention définie à l'aide des revendications annexées.

Les exemples suivants illustrent l'invention sans la limiter.

### Exemple 1 : Composition à base de DMDS à odeur masquée

Afin de caractériser une composition parfumante permettant de masquer, d'améliorer, l'odeur du DMDS, une procédure de test olfactif a été mise au point. Cette procédure permet de classer différentes formulations de manière hédonique.

### Conditions opératoires :

Pour réaliser ce test olfactif, on utilise des fûts en polyéthylène (PE) de 30 litres, chacun muni d'un couvercle dans lequel est découpée une trappe d'environ 10 cm x 10 cm, permettant à un opérateur (panéliste) de sentir les vapeurs contenues dans le fût.

Dans chacun des fûts est placé un cristallisoir contenant 2 feuilles de papier absorbant (papier chromatographique). Sur chaque feuille est versé 1 mL de composition à tester. Les fûts sont conservés fermés pendant 24 heures à température ambiante. L'évaluation est réalisée ensuite en aveugle.

Les panélistes, au nombre de 10, viennent à tour de rôle pour tester quelques produits par séance (au maximum 3 produits par séance). Ils commencent par sentir le fût dans lequel se trouve le DMDS de référence de cette étude, puis une des compositions à tester.

Les panélistes attribuent, selon leur préférence, une note à chacune des compositions à tester, par rapport à la référence qui a reçu arbitrairement la note 5. Les notes données par les panélistes vont de 1 (produit le plus agréable) à 10 (produit le plus désagréable).

### Préparation des échantillons de test :

Le DMDS, sans agent masquant d'odeur, est un DMDS industriel produit par Arkema, présente une pureté supérieure à 99,7% et contient moins de 100 ppm de méthylmercaptan et moins de 50 ppm de sulfure de diméthyle.

À ce DMDS sont ajoutés 3000 ppm d'une composition parfumante ayant la composition suivante : 25% acétate d'iso-amyle, 50% *ortho*-phtalate de diéthyle, 15% butyrate de 2-méthylbutyle, 10% acétate de benzyle comme décrit dans le brevet EP 0 976 726. Cet échantillon est l'échantillon de référence pour le test olfactif et est nommé : A₁.

Au même DMDS industriel produit par Arkema, sans agent masquant d'odeur sont ajoutés 3000 ppm de la composition parfumante C*ᵢ* selon l'invention et précédemment définie. Cet échantillon est nommé : A₂.

### Résultats :

Les résultats du test olfactif sont reproduits dans le tableau 1 suivant :

**-- Tableau 1 --**

| ***Échantillon à tester*** | ***Moyenne*** | ***Écart-type*** | ***Groupe*** |
|---|---|---|---|
| A₁ | 5 | 0 | A |
| A₂ | 2,83 | 0,96 | B |

Le traitement statistique de ces résultats permet de calculer l'écart-type et de classer les échantillons en deux groupes en étudiant la PPDS (plus petite différence significative) donnée dans ce test à 0,87.

Le test PPDS est un test statistique de comparaison de moyennes et permet de déterminer si les moyennes de 2 échantillons sont significativement différentes ou non, d'un point de vue statistique.

Dans les exemples de la présente invention, le paramétrage statistique utilisé est fixé à 95%. Si les moyennes ne sont pas significativement différentes, les 2 échantillons sont classés dans un même groupe. Si les moyennes sont significativement différentes, les 2 échantillons constituent 2 groupes séparés (A et B dans les exemples illustratifs de l'invention).

La même opération est effectuée pour comparer tous les échantillons, ce qui permet *in fine* d'arriver à 1, 2 ou plusieurs groupes, chacun constitué d'échantillons dont les notes moyennes ne sont pas significativement différentes. Ces différents traitements sont réalisés à l'aide du logiciel FIZZ version 2.01 (Biosystèmes, Couternon, France).

Il y a donc une différence statistique très significative indiquant une perception de l'odeur nettement plus agréable de l'échantillon A₂ que de l'échantillon A₁.

### Exemple 2 : Composition à base de DMSO à odeur masquée

Un test olfactif similaire à celui décrit dans l'exemple 1 est réalisé en prenant pour base du DMSO au lieu de DMDS.

### Préparation des échantillons de test :

Le DMSO de référence est un DMSO industriel de pureté égale à 99.97% produit par Arkema, puis additivé de 50 ppm de sulfure de diméthyle (DMS). Cet échantillon est nommé B₁.

Le même lot de DMSO additivé de 50 ppm de sulfure de diméthyle est additionné de 700 ppm de la composition parfumante C*ᵢ* selon l'invention. Cet échantillon est nommé B₂.

Les résultats du test olfactif sont reproduits dans le tableau 2 suivant :

**-- Tableau 2 --**

| ***Échantillon à tester*** | ***Moyenne*** | ***Écart-type*** | ***Groupe*** |
|---|---|---|---|
| B₁ | 5,93 | 1.33 | A |
| B₂ | 2,75 | 1.52 | B |

Comme pour l'exemple 1, le traitement statistique de ces résultats permet de calculer l'écart-type et de classer les échantillons en deux groupes en étudiant la PPDS (plus petite différence significative) donnée dans ce test à 1,01.

Il y a donc une différence statistique très significative indiquant une perception de l'odeur nettement plus agréable de l'échantillon B₂ que de l'échantillon B₁.

### Exemple 3 : Composition à base de DMSO à odeur masquée

Le même DMSO industriel que celui de l'exemple 2, de pureté 99.97% produit par Arkema, est testé sans ajout des 50 ppm de DMS, suivant le test olfactif décrit à l'exemple 2. Cet échantillon est nommé C₁.

À ce même DMSO industriel est additionné 150 ppm de la composition parfumante C*ᵢ* selon l'invention. Cet échantillon est nommé C₂.

Les résultats du test olfactif sur C₁ et C₂ indiquent que l'échantillon C₂ est jugé statistiquement nettement plus agréable que l'échantillon C₁.

## Revendications

1. Composition comprenant :
a) au moins un sulfure organique, éventuellement sous forme oxyde, de formule générale (1) : 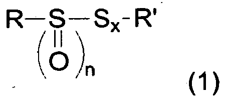 dans laquelle R est choisi parmi un radical alkyle, linéaire ou ramifié, contenant de 1 à 4 atomes de carbone, et un radical alcényle, linéaire ou ramifié, contenant de 2 à 4 atomes de carbone ; n est égal à 0, 1 ou 2 ; x est un nombre entier choisi parmi 0, 1, 2, 3 ou 4, de préférence x représente 1, 2, 3 ou 4 ; R' est choisi parmi un radical alkyle, linéaire ou ramifié, contenant de 1 à 4 atomes de carbone, et un radical alcénylène, linéaire ou ramifié, contenant de 2 à 4 atomes de carbone ou, seulement lorsque n = x = 0, un atome d'hydrogène ; et
b) au moins un agent masquant d'odeur comprenant au moins un mono-ester, au moins un di- et/ou tri-ester, au moins un alcool, au moins une cétone et éventuellement au moins un terpène.

2. Composition selon la revendication 1, dans laquelle le composant a) est choisi parmi le disulfure de diméthyle et le diméthylsulfoxyde.

3. Composition selon la revendication 1 ou 2, dans laquelle l'agent masquant d'odeur comprend :
b1) de 1 % à 40% en poids d'au moins un mono-ester ;
b2) de 10% à 70% en poids d'au moins un di- et/ou tri-ester ;
b3) de 1 % à 30% **en poids** d'au moins un alcool ;
b4) de 0,5% à 20% **en poids** d'au moins une cétone de formule R^{a}-CO-R^{b}, dans laquelle R^{a} représente une chaîne hydrocarbonée comportant de 1 à 6 atomes de carbone, linéaire ou ramifié, éventuellement comprenant une ou plusieurs insaturation(s) sous forme de double(s) liaison(s), et R^{b} représente une chaîne hydrocarbonée cyclique ou bien une chaîne hydrocarbonée linéaire ou ramifiée éventuellement mais de préférence substituée par une structure cyclique, R^{b} comportant de 6 à 12 atomes de carbone, comprenant éventuellement une ou plusieurs insaturation(s) sous forme de double(s) liaison(s) et étant éventuellement substitué par un ou plusieurs groupes hydroxyle ; et
b5) éventuellement jusqu'à 20% en poids d'au moins un terpène.

4. Composition selon la revendication 3, dans laquelle l'agent masquant d'odeur b) comprend de 1% à 40%, de préférence de 2% à 35%, de préférence encore de 5% à 30% en poids, par rapport au poids total de la composition, d'au moins un mono-ester b1), choisi parmi les esters d'acides en C₂-C₂₀ saturés ou insaturés, tels que les acétates, propionates, butyrates, méthylbutyrates, pentanoates, hexanoates, heptanoates, caproates, oléates, linoléates, linolénates, d'éthyle, de propyle, de butyle, de pentyle, de 2-méthylbutyle, d'iso-amyle, d'hexyle, de benzyle, de phényléthyle, de menthyle, de carvyle, et autres, ainsi que leurs mélanges.

5. Composition selon la revendication 3, dans laquelle l'agent masquant d'odeur b) comprend au moins un di- et/ou tri-ester b2), en une quantité comprise entre 10% et 70%, de préférence entre 15% et 65%, de préférence encore entre 20% et 60% en poids, choisi(s) parmi les *ortho*-phtalates, (de préférence l'*ortho-*phtalate de diéthyle), les citrates (de préférence le citrate de triéthyle) et les malonates (de préférence le malonate de diéthyle).

6. Composition selon la revendication 3, dans laquelle l'agent masquant d'odeur b) comprend de 1 % à 30%, de préférence de 5% à 25% en poids par rapport au poids total de la composition b), d'au moins un alcool b3), avantageusement au moins un mono-alcool comprenant de 1 à 30 atomes des carbone, de préférence de 6 à 20 atomes de carbone, de préférence encore de 8 à 11 atomes de carbone, lesdits atomes de carbone formant une chaîne linéaire ou ramifiée comportant éventuellement une ou plusieurs insaturation(s) sous forme de double(s) liaison(s), et comportant éventuellement une structure cyclique à 5 ou 6 chaînons, saturée, ou totalement ou partiellement insaturée.

7. Composition selon la revendication 6, dans laquelle l'alcool est choisi parmi le menthol, le néo-menthol, l'alcool phényléthylique, l'alcool benzylique, le citronellol, le dihydromyrcénol, le dihydrotérpinéol, le dimétol, l'éthyl-linalol, le géraniol, le linalol, le tétrahydrolinalol, le tétrahydromyrcénol, le nérol,
ainsi que les mélanges de deux ou plusieurs d'entre eux.

8. Composition selon la revendication 3, dans laquelle l'agent masquant d'odeur b) comprend entre 0,5% et 20%, de préférence entre 1 % et 10% en poids par rapport au poids total de la composition b), d'au moins une cétone b4) choisie(s) parmi les damascones, les damascénones, les ionones, les irisones, les méthylionones, et la frambinone, ainsi que les mélanges de celles-ci.

9. Composition selon la revendication 3, dans laquelle l'agent masquant d'odeur b) comprend éventuellement jusqu'à 20%, de préférence de 1 % à 10% en poids par rapport au poids total de la composition, d'au moins un terpène b5), choisi(s) parmi les terpinènes, le myrcène, le limonène, le terpinolène, les pinènes, le sabinène, le camphène, les mélanges de deux ou plusieurs d'entre eux, ainsi que les essences à base de terpènes, notamment celles comprenant ces ingrédients.

10. Composition selon l'une quelconque des revendications précédentes, dans laquelle l'agent masquant d'odeur comprend en poids :
- de 5% à 30% en poids d'au moins un mono-ester b1), choisi parmi l'acétate d'*iso-*amyle, le méthyl-2-butyrate d'ethyle, le butyrate d'iso-amyle, l'acétate de phényléthyle, le caproate d'éthyle, l'acétate de benzyle, l'acétate d'hexyle et leurs mélanges ;
- de 20% à 60% en poids d'au moins un di- et/ou tri-ester b2) choisi(s) parmi les *ortho*-phtalates (de préférence l'*ortho*-phtalate de diéthyle), les citrates (de préférence le citrate de triéthyle) et les malonates (de préférence le malonate de diéthyle), et leurs mélanges ;
- de 5% à 25% **en poids** d'au moins un alcool, de préférence d'au moins deux alcools, de préférence encore d'au moins trois alcools, b3) ;
- de 1% à 10% **en poids** d'au moins une cétone, de préférence au moins deux cétones, de préférence encore au moins trois cétones b4) ; et
- de 1% à 10% **en poids** d'au moins un, de préférence au moins deux, de préférence un mélange de terpènes b5).

11. Composition selon l'une quelconque des revendications précédentes, dans laquelle l'agent masquant b) est présent en une quantité allant de 10 ppm, à 2%, de préférence de 10 ppm à 1 % en poids par rapport au poids total de la composition.

12. Composition selon l'une quelconque des revendications précédentes, dans laquelle le sulfure organique a) est le disulfure de diméthyle et la quantité d'agent masquant d'odeur b) est comprise entre 0,001 % et 0,5% en poids par rapport au poids total de la composition, et plus particulièrement comprise entre 0,1% et 0,5% en poids, avantageusement égale à 0,3%.

13. Composition selon l'une quelconque des revendications précédentes, dans laquelle le sulfure organique a) est le diméthylsulfoxyde et la quantité d'agent masquant d'odeur b) est comprise entre 0,001 % et 0,2% en poids par rapport au poids total de la composition, de préférence comprise entre 100 ppm et 1000 ppm, par exemple 500 ppm en poids.

## Patentansprüche

1. Zusammensetzung, umfassend:
a) mindestens ein organisches Sulfid, gegebenenfalls in Oxidform, der allgemeinen Formel (1): 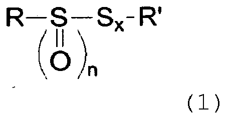 worin R aus einem linearen oder verzweigten Alkylrest mit 1 bis 4 Kohlenstoffatomen und einem linearen oder verzweigten Alkenylrest mit 2 bis 4 Kohlenstoffatomen ausgewählt ist; n gleich 0, 1 oder 2 ist; x eine ganze Zahl ist, die aus 0, 1, 2, 3 oder 4 ausgewählt ist, und vorzugsweise für 1, 2, 3 oder 4 steht; R' aus einem linearen oder verzweigten Alkylrest mit 1 bis 4 Kohlenstoffatomen und einem linearen oder verzweigten Alkenylrest mit 2 bis 4 Kohlenstoffatomen oder nur dann, wenn n = x = 0, einem Wasserstoffatom ausgewählt ist; und
b) mindestens ein geruchsmaskierendes Mittel, umfassend mindestens einen Monoester, mindestens einen Di- und/oder Triester, mindestens einen Alkohol, mindestens ein Keton und gegebenenfalls mindestens ein Terpen.

2. Zusammensetzung nach Anspruch 1, wobei Komponente a) aus Dimethyldisulfid und Dimethylsulfoxid ausgewählt ist.

3. Zusammensetzung nach Anspruch 1 oder 2, wobei das geruchsmaskierende Mittel:
b1) 1 bis 40 Gew.-% mindestens eines Monoesters;
b2) 10 bis 70 Gew.-% mindestens eines Di- und/oder Triesters;
b3) 1 bis 30 Gew.-% mindestens eines Alkohols;
b4) 0,5 bis 20 Gew.-% mindestens eines Ketons der Formel R^{a}-CO-R^{b}, worin R^{a} für eine lineare oder verzweigte Kohlenwasserstoffkette mit 1 bis 6 Kohlenstoffatomen und gegebenenfalls einer oder mehreren Ungesättigtheiten in Form einer oder mehrerer Doppelbindungen steht und R^{b} für eine cyclische Kohlenwasserstoffkette oder auch eine lineare oder verzweigte Kohlenwasserstoffkette, die gegebenenfalls, aber vorzugsweise durch eine cyclische Struktur substituiert ist, wobei R^{b} 6 bis 12 Kohlenstoffatome enthält, gegebenenfalls eine oder mehrere Ungesättigtheiten in Form einer oder mehrerer Doppelbindungen umfasst und gegebenenfalls durch eine oder mehrere Hydroxylgruppen substituiert ist, steht; und
b5) gegebenenfalls bis zu 20 Gew.-% mindestens eines Terpens
umfasst.

4. Zusammensetzung nach Anspruch 3, wobei das geruchsmaskierende Mittel b) 1 bis 40 Gew.-%, vorzugsweise 2 bis 35 Gew.-%, weiter bevorzugt 5 bis 30 Gew.-%, bezogen auf das Gesamtgewicht der Zusammensetzung, mindestens eines Monoesters b1), der aus Estern von gesättigten oder ungesättigten C₂-C₂₀-Säuren, wie Essigsäure-, Propionsäure-, Buttersäure-, Methylbuttersäure-, Pentansäure-, Hexansäure-, Heptansäure-, Capronsäure-, Ölsäure-, Linolsäure- und Linolensäureethylestern, -propylestern, -butylestern, -pentylestern, -2-methyl-butylestern, -isoamylestern, -hexylestern, -benzylestern, -phenylethylestern, -menthylestern oder -carvylestern und dergleichen, sowie Mischungen davon ausgewählt ist, umfasst.

5. Zusammensetzung nach Anspruch 3, wobei das geruchsmaskierende Mittel b) mindestens einen Di - und/oder Triester b2) in einer Menge zwischen 10 und 70 Gew.-%, vorzugsweise zwischen 15 und 65 Gew.-%, weiter bevorzugt zwischen 20 und 60 Gew.-%, der aus ortho-Phthalaten (vorzugsweise Diethyl-ortho-phthalat), Citraten (vorzugsweise Triethylcitrat) und Malonaten (vorzugsweise Diethylmalonat) ausgewählt ist, umfasst.

6. Zusammensetzung nach Anspruch 3, wobei das geruchsmaskierende Mittel b) 1 bis 30 Gew.-%, vorzugsweise 5 bis 25 Gew.-%, bezogen auf das Gesamtgewicht der Zusammensetzung b), mindestens eines Alkohols b3), vorteilhafterweise mindestens eines Monoalkohols mit 1 bis 30 Kohlenstoffatomen, vorzugsweise 6 bis 20 Kohlenstoffatomen und weiter bevorzugt 8 bis 11 Kohlenstoffatomen, wobei die Kohlenstoffatome eine lineare oder verzweigte Kette bilden, die gegebenenfalls eine oder mehrere Ungesättigtheiten in Form einer oder mehrerer Doppelbindungen umfasst und gegebenenfalls eine 5 - oder 6-gliedrige cyclische Struktur, die gesättigt oder vollständig oder teilweise ungesättigt ist, umfasst, umfasst.

7. Zusammensetzung nach Anspruch 6, wobei der Alkohol aus Menthol, neo-Menthol, Phenylethylalkohol, Benzylalkohol, Citronellol, Dihydromyrcenol, Dihydroterpineol, Dimetol, Ethyllinalol, Geraniol, Linalol, Tetrahydrolinalol, Tetrahydromyrcenol, Nerol sowie Mischungen von zwei oder mehr davon ausgewählt ist.

8. Zusammensetzung nach Anspruch 3, wobei das geruchsmaskierende Mittel b) zwischen 0,5 und 20 Gew.-%, vorzugsweise zwischen 1 und 10 Gew.-%, bezogen auf das Gesamtgewicht der Zusammensetzung b), mindestens eines Ketons b4), das aus Damasconen, Damascenonen, Iononen, Irisonen, Methyliononen und Frambinon sowie Mischungen davon ausgewählt ist, umfasst.

9. Zusammensetzung nach Anspruch 3, wobei das geruchsmaskierende Mittel b) gegebenenfalls bis zu 20 Gew.-%, vorzugsweise 1 bis 10 Gew.-%, bezogen auf das Gesamtgewicht der Zusammensetzung, mindestens eines Terpens b5), das aus Terpinenen, Myrcen, Limonen, Terpinolen, Pinenen, Sabinen, Camphen, Mischungen von zwei oder mehr davon sowie Essenzen auf Basis von Terpenen, insbesondere denjenigen, die diese Bestandteile umfassen, ausgewählt ist, umfasst.

10. Zusammensetzung nach einem der vorhergehenden Ansprüche, wobei das geruchsmaskierende Mittel:
- 5 bis 30 Gew.-% mindestens eines Monoesters b1), der aus Essigsäureisoamylester, 2-Methylbuttersäureethylester, Buttersäureisoamylester, Essigsäurephenylethylester, Capronsäureethylester, Essigsäurebenzylester, Essigsäurehexylester und Mischungen davon ausgewählt ist;
- 20 bis 60 Gew.-% mindestens eines Di- und/oder Triesters b2), der aus ortho-Phthalaten (vorzugsweise Diethyl-ortho-phthalat), Citraten (vorzugsweise Triethylcitrat) und Malonaten (vorzugsweise Diethylmalonat) und Mischungen davon ausgewählt ist;
- 5 bis 25 Gew.-% mindestens eines Alkohols, vorzugsweise mindestens zweier Alkohole, weiter bevorzugt mindestens dreier Alkohole, b3);
- 1 bis 10 Gew.-% mindestens eines Ketons, vorzugsweise mindestens zweier Ketone, weiter bevorzugt mindestens dreier Ketone, b4); und
- 1 bis 10 Gew.-% mindestens eines Terpens, vorzugsweise mindestens zweier Terpene, vorzugsweise einer Mischung von Terpenen, b5) umfasst.

11. Zusammensetzung nach einem der vorhergehenden Ansprüche, wobei das Maskierungsmittel b) in einer Menge im Bereich von 10 Gew.-ppm bis 2 Gew.-%, vorzugsweise 10 Gew.-ppm bis 1 Gew.-%, bezogen auf das Gesamtgewicht der Zusammensetzung, vorliegt.

12. Zusammensetzung nach einem der vorhergehenden Ansprüche, wobei es sich bei dem organischen Sulfid a) um Dimethyldisulfid handelt und die Menge des geruchsmaskierenden Mittels b) zwischen 0,001 und 0,5 Gew.-%, bezogen auf das Gesamtgewicht der Zusammensetzung, und spezieller zwischen 0,1 und 0,5 Gew.-% liegt und vorteilhafterweise gleich 0,3% ist.

13. Zusammensetzung nach einem der vorhergehenden Ansprüche, wobei es sich bei dem organischen Sulfid a) um Dimethylsulfoxid handelt und die Menge des geruchsmaskierenden Mittels b) zwischen 0,001 und 0,2 Gew.-%, bezogen auf das Gesamtgewicht der Zusammensetzung, vorzugsweise zwischen 100 und 1000 Gew.-ppm, beispielsweise 500 Gew.-ppm, liegt.

## Claims

1. Composition comprising:
a) at least one organic sulphide, optionally in oxide form, of general formula (1): 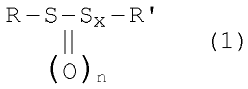 in which R is chosen from a linear or branched alkyl radical containing from 1 to 4 carbon atoms, and a linear or branched alkenyl radical containing from 2 to 4 carbon atoms; n is equal to 0, 1 or 2; x is an integer chosen from 0, 1, 2, 3 or 4, preferably x represents 1, 2, 3 or 4; R' is chosen from a linear or branched alkyl radical containing from 1 to 4 carbon atoms, and a linear or branched alkenylene radical containing from 2 to 4 carbon atoms or, only when n = x = 0, a hydrogen atom; and
b) at least one odour-masking agent comprising at least one monoester, at least one di- and/or triester, at least one alcohol, at least one ketone and, optionally, at least one terpene.

2. Composition according to Claim 1, in which the component a) is chosen from dimethyl disulphide and dimethyl sulphoxide.

3. Composition according to Claim 1 or 2, in which the odour-masking agent comprises:
b1) from 1% to 40% by weight of at least one monoester;
b2) from 10% to 70% by weight of at least one di- and/or triester;
b3) from 1% to 30% by weight of at least one alcohol;
b4) from 0.5% to 20% by weight of at least one ketone of formula R^{a}-CO-R^{b}, in which R^{a} represents a linear or branched hydrocarbon-based chain containing from 1 to 6 carbon atoms, optionally comprising one or more unsaturation(s) in the form of one or more double bond(s), and R^{b} represents a cyclic hydrocarbon-based chain or else a linear or branched hydrocarbon-based chain optionally, but preferably, substituted with a cyclic structure, R^{b} containing from 6 to 12 carbon atoms, optionally comprising one or more unsaturation(s) in the form of one or more double bond(s) and being optionally substituted with one or more hydroxyl groups; and
b5) optionally, up to 20% by weight of at least one terpene.

4. Composition according to Claim 3, in which the odour-masking agent b) comprises from 1% to 40%, preferably from 2% to 35%, more preferably from 5% to 30% by weight, relative to the total weight of the composition, of at least one monoester b1), chosen from saturated or unsaturated C₂-C₂₀ acid esters, such as ethyl, propyl, butyl, pentyl, 2-methylbutyl, isoamyl, hexyl, benzyl, phenylethyl, menthyl or carvyl acetates, propionates, butyrates, methylbutyrates, pentanoates, hexanoates, heptanoates, caproates, oleates, linoleates and linolenates, and the like,
and also mixtures thereof.

5. Composition according to Claim 3, in which the odour-masking agent b) comprises at least one di- and/or triester b2), in an amount of between 10% and 70%, preferably between 15% and 65%, more preferably between 20% and 60% by weight, chosen from ortho-phthalates (preferably diethyl ortho-phthalate), citrates (preferably triethyl citrate) and malonates (preferably diethyl malonate).

6. Composition according to Claim 3, in which the odour-masking agent b) comprises from 1% to 30%, preferably from 5% to 25% by weight relative to the total weight of the composition b), of at least one alcohol b3), advantageously at least one monoalcohol containing from 1 to 30 carbon atoms, preferably from 6 to 20 carbon atoms, more preferably from 8 to 11 carbon atoms, said carbon atoms forming a linear or branched chain optionally comprising one or more unsaturation(s) in the form of one or more double bond(s), and optionally comprising a 5- or 6-membered cyclic structure which is saturated or completely or partially unsaturated.

7. Composition according to Claim 6, in which the alcohol is chosen from menthol, neomenthol, phenylethyl alcohol, benzyl alcohol, citronellol, dihydromyrcenol, dihydroterpineol, dimetol, ethyllinalol, geraniol, linalol, tetrahydrolinalol, tetrahydromyrcenol, nerol, and also mixtures of two or more thereof.

8. Composition according to Claim 3, in which the odour-masking agent b) comprises between 0.5% and 20%, preferably between 1% and 10% by weight relative to the total weight of the composition b), of at least one ketone b4) chosen from damascones, damascenones, ionones, irisones, methylionones and frambinone, and also mixtures thereof.

9. Composition according to Claim 3, in which the odour-masking agent b) optionally comprises up to 20%, preferably from 1% to 10% by weight relative to the total weight of the composition, of at least one terpene b5), chosen from terpinenes, myrcene, limonene, terpinolene, pinenes, sabinene, camphene, mixtures of two or more thereof, and also essences based on terpenes, in particular those comprising these ingredients.

10. Composition according to any one of the preceding claims, in which the odour-masking agent comprises by weight:
- from 5% to 30% by weight of at least one monoester b1), chosen from isoamyl acetate, ethyl 2-methyl butyrate, isoamyl butyrate, phenylethyl acetate, ethyl caproate, benzyl acetate, hexyl acetate and mixtures thereof;
- from 20% to 60% by weight of at least one di- and/or triester b2) chosen from ortho-phthalates (preferably diethyl ortho-phthalate), citrates (preferably triethyl citrate) and malonates (preferably diethyl malonate), and mixtures thereof;
- from 5% to 25% by weight of at least one alcohol, preferably of at least two alcohols, more preferably of at least three alcohols, b3);
- from 1% to 10% by weight of at least one ketone, preferably at least two ketones, more preferably at least three ketones, b4); and
- from 1% to 10% by weight of at least one, preferably at least two, preferably a mixture of, terpene(s), b5).

11. Composition according to any one of the preceding claims, in which the masking agent b) is present in an amount ranging from 10 ppm to 2%, preferably from 10 ppm to 1% by weight relative to the total weight of the composition.

12. Composition according to any one of the preceding claims, in which the organic sulphide a) is dimethyl disulphide and the amount of odour-masking agent b) is between 0.001% and 0.5% by weight relative to the total weight of the composition, and more particularly between 0.1% and 0.5% by weight, advantageously equal to 0.3%.

13. Composition according to any one of the preceding claims, in which the organic sulphide a) is dimethyl sulphoxide and the amount of odour-masking agent b) is between 0.001% and 0.2% by weight relative to the total weight of the composition, preferably between 100 ppm and 1000 ppm, for example 500 ppm by weight.
